# EUROPEAN PATENT SPECIFICATION

(11) **EP 2 377 522 B1**
(45) Date of publication and mention of the grant of the patent: **16.01.2013**
(21) Application number: 11162434.2
(22) Date of filing: 14.04.2011
(51) Int. Cl.: A61K 9/20, A61K 31/55

(54) **Orally disintegrating tablet formulations of mirtazapine and process for preparing the same**
Formulierungen von im Mund zerfallenden Mirtazapintabletten und Herstellungsverfahren dafür.
Formulations de comprimé à désintégration orale de mirtazapine et procédé de préparation.

(30) Priority: 15.04.2010 TR 201002942
(43) Date of publication of application: 19.10.2011
(73) Proprietor: Sanovel Ilaç Sanayi Ve Ticaret Anonim Sirketi, 34460 Sariyer/Istanbul (TR)
(72) Inventor: Cifter, Ümit, 34398, Istanbul (TR); Türkyilmaz, Ali, 34398, Istanbul (TR); Turp, Hasan Ali, 34398, Istanbul (TR); Yelken, Gülay, 34398, Istanbul (TR)
(74) Representative: Sevinç, Erkan

(56) References cited:
- EP-B1- 1 223 914
- WO-A1-99/44580
- WO-A1-2005/034921
- WO-A2-2006/092812
- DE-A1-102004 034 043

## Description

### Technical Field of the Invention

The present invention relates to silicon dioxide free orally disintegrating tablet formulations of mirtazapine or a pharmaceutically acceptable salt thereof comprising crospovidone and sodium stearyl fumarate and one or more pharmaceutically acceptable excipient and process for preparing such a formulation. More specifically, the weight ratio of crospovidone and sodium stearyl fumarate is between 1:25 to 25:1 (w/w).

### Background of the Invention

Mirtazapine has a tetracyclic chemical structure and belongs to the piperazino-azepine group of compounds, which is known as 1,2,3,4,10,14b-hexahydro-2-methylpyrazino [2,1-a] pyrido [2,3-c] benzazepine, and its chemical structure is shown in the Formula I.

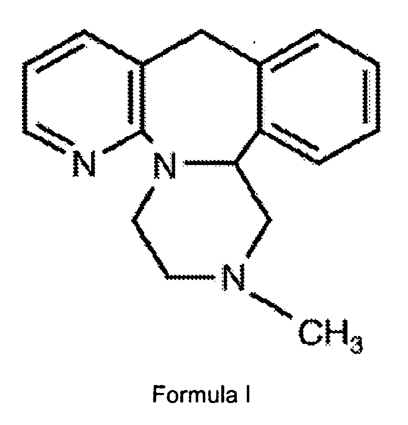

Mirtazapine is indicated for the treatment of major depressive disorder and available for oral administration in conventional tablet formulations and orally disintegrating tablet formulations containing 15, 30 or 45 mg of mirtazapine.

Orally disintegrating formulations are becoming an increasingly important issue in the area of better patient compliance comparative to the conventional solid dosage forms for oral administration such as capsules and tablets, which are the most commonly used. In particular pediatric and geriatric patients, and patients with mental problems such as depression, often experience difficulties in swallowing solid dosage forms. Besides, conventional solid dosage forms are not suitable for bedridden or busy and travelling patients, in case the patient may not have easy access to water. Thus, orally disintegrating compositions represent an alternative for such patients and provide for a better patient compliance with recommended pharmaceutical therapies.

Besides, the orally disintegrating dosage form is one of the advantageous methods to deliver these drugs to such patients. By administering the orally disintegrating dosage forms, faster absorption of the drug occurs through buccal mucosa and it may reduce the first pass metabolism leading to better efficacy of the drug. This dosage form enhances the clinical effects of some drugs by leading to an increase in bioavailability and a reduction in side effects because of avoidance of first-pass liver metabolism.

Various formulations and methods are already known for the preparation of orally disintegrating formulations of mirtazapine or pharmaceutically acceptable salts thereof. However, to develop orally disintegrating compositions are difficult because of several different reasons. A satisfied orally disintegrating dosage form needs to meet number of requirements. Firstly, it has to disintegrate in the oral cavity rapidly. Moreover, a premature release in the mouth could also lead to problems due to the often unpleasant taste of the active ingredient. Besides, these compositions should be very porous and should not be very hard. These porous compositions tend to be very sensitive to humidity. As a consequence, they may have some stability problems. Finally, any orally disintegrating composition with suitable organoleptic and pharmacokinetic properties must also be manufactured at commercially useful rates and yields.

To fulfill all these requirements the formulation for a specific drug needs to be adapted in particular by a careful selection of the excipients used. However, the excipients selected may lead to formulations which are not bioavailable to the corresponding conventional dosage forms. Thus, they have to be chosen very carefully. Additionally, precautions have to be taken at the preparation, packaging, handling and storing of the finished dosage forms of orally disintegrating compositions since they tend to be both hygroscopic and friable.

Thus, various technologies have been developed which enable the preparation of compositions that disintegrate quickly in the oral cavity. These technologies are including spray drying, freeze drying and floss formation. However, all these technologies have their own limitations and have very complicated manufacturing processes.

The spray drying technique involves spraying the drug and excipients into a chamber maintained at a high temperature. As a result this technique is not suitable for application to thermo- labile drugs. Additionally, the spray drying technology leads only to a very poor output and is very expensive.

Freeze drying on a large scale has not been found to be very effective. Moreover, it has limitations due to factors such as time, costly equipment and processing conditions. Besides, the Zydis^{®} tablets prepared by this technique are so fragile that the formation of the matrix material has to take place in a specific container. Tablets manufactured by this technology require a special type of packaging and careful handling during dispensing and administration to the patients, since they are prone to breakage. Such US Patents which disclose these formulations are US 4 642 903, 5 188 825, 5 631 023, 5 827 541 and 5 976 577.

The floss formation technique includes compressing micro- particles of a drug and a cotton candy-like fibrous saccharide matrix, such as sucrose, dextrose, lactose and fructose. It is also known as Flash Dose technology (Fuisz) and requires specific equipment for making the specific matrix, which is sensitive to moisture, and generally results in tablets of high friability.

EP 1 223 914 B1 discloses a dosage unit for peroral administration, comprising a pharmaceutical formulation of mirtazapine as an active substance with a polymer layer which is made from methylaminoethyl-methacrylate and neutral methacrylic ester monomers and. However, it is a disadvantage that the preparation of these specific polymers and coating requires special equipment and processes.

Finally, many of these techniques have proved to be successful only for specific drugs and are often not transferable to other active ingredients.

Thus, a need rises for orally disintegrating tablet formulations of mirtazapine or a pharmaceutically acceptable salt thereof and a process for preparing such formulation which overcomes the above described problems in prior art and having additive advantages over them. Further advantages and embodiments of the present invention will become apparent from the following description.

### Detailed Description of the Invention

The main object of the present invention is to provide an improved orally disintegrating tablet formulation of mirtazapine or a pharmaceutically acceptable salt thereof which overcomes above described problems with using adequate excipients and which further provide the advantageous property of allowing the active medicament to disintegrate rapidly in the oral cavity without remaining substantial amounts of the active ingredient and which have a pleasant mouth feel.

Another object of the present invention is to provide a simple, cost-effective and time saving process for the preparation of orally disintegrating tablet formulation of mirtazapine or a pharmaceutically acceptable salt thereof.

Yet another object of the present invention is to provide an orally disintegrating tablet formulation of mirtazapine or a pharmaceutically acceptable salt thereof which has good mechanical strength enough to be processed in high speed tableting machines and shipped in low cost packages.

A further object of the present invention is to provide bioavailable and stable orally disintegrating tablet formulation of mirtazapine or a pharmaceutically acceptable salt thereof throughout the shelf-life. According to this object, in this invention an orally disintegrating tablet formulation of mirtazapine or a pharmaceutically acceptable salt thereof which is comparable with the existing conventional solid dosage forms is provided, however unexpected benefits are found with oral disintegration. Because, presentation of mirtazapine in liquid or in conventional solid or liquid oral dosage forms, having their own limitations, are not ideal for use in pediatric or geriatric patients or in patients suffering from from depression.

According to one of the main objects of the present invention is to provide an orally disintegrating tablet formulation of mirtazapine or a pharmaceutically acceptable salt thereof without using silicon dioxides. It is known that an improved orally disintegrating tablet formulation should have minimum grift or sandy effect, and to provide this effect silicon dioxide is mostly used. However, silicon dioxides, especially colloidal silicon dioxide is very hygroscopic and this cause problems when preparing the orally disintegrating tablet formulations. Thus, precautions have to be taken at the preparation, packaging, handling and storing of the finished dosage forms of orally disintegrating formulations since they tend to be both hygroscopic and friable. We have surprisingly obtained good and improved results despite not using silicon dioxide, especially colloidal silicon dioxide. Thus, this is achieved by the adequate selection of excipients which will be further detailed below.

According to this object the present invention is directed to a silicon dioxide free orally disintegrating tablet formulation of mirtazapine or a pharmaceutically acceptable salt thereof comprising crospovidone and sodium stearyl fumarate in a weight ratio of between 1:25 to 25:1 (w/w) and one or more pharmaceutically acceptable excipient.

Without using silicon dioxide, the selection of excipients has more importance to obtain the ideal disintegrating time during the shelf life. Thus, crospovidone has physical and chemical properties that make it ideal for constituting the appropriate disintegrant for this invention. Because crospovidone particles have a very different appearance from those of the other disintegrants. Crospovidone particles seem to consist of aggregates of smaller particles that are fused together. This aggregation gives crospovidone a spongy, highly porous appearance and it swells very little, yet takes water into its network quite rapidly. This helps crospovidone to dissolve easily and quickly in a little amount of water or saliva and makes its disintegrating rate much faster than other related excipients.

Another object of the invention is to provide an orally disintegrating tablet formulation of mirtazapine or a pharmaceutically acceptable salt thereof which does not comprise magnesium stearate. In prior art, most commonly used excipient in orally disintegrating tablet formulations with mirtazapine as lubricant, is magnesium stearat. It is known that magnesium stearate has some disadvantages despite being a good lubricant and because of this it is used in small quantities during drug manufacturing process. Magnesium stearate is practically insoluble in water and because of this hydrophobic characteristic it may retard the disintegration time. Another disadvantage of magnesium stearate is found in manufacturing process which is the blending time. Blending time should be limited. Long blending times can result in the formulation of hydrophobic powder beds that do not disperse easily and overblending can cause compaction problems. Tablet disintegration time increased and crushing strength decreased as the time of blending increased; and magnesium stearate may also increase tablet friability. Sodium stearyl fumarate is extremely effective lubricant and less hydrophobic than magnesium stearate. It improves the wettebality of the tablet ingredients, resulting in shorter disintegration times. Sodium stearyl fumarate also doesn't have the over blending problems seen with magnesium steare.

Besides, sodium stearyl fumarate can perform more than one function in the formulation of this present invention. For example it can function as both lubricant and a stabilizer. This helps the formulation to stay stable throughout the shelf-life. It has surprisingly been found that the specific combination of crospovidone and sodium stearyl fumarate with the active ingredient mirtazapine or a pharmaceutically acceptable salt thereof results a synergistic effect over the disintegration time, stability and manufacturing process. According to this embodiment, the weight ratio of crospovidone to sodium stearyl fumarate is in between 1:25 to 25:1 (w/w), preferably in between 1:1 to 15:1 (w/w), more preferably in between 5:1 to 10:1 (w/w).

More specifically, the present invention is related to colloidal silicon dioxide free orally disintegrating tablet formulation of mirtazapine or a pharmaceutically acceptable salt thereof comprising crospovidone and sodium stearyl fumarate in a weight ratio of between 1:25 to 25:1 (w/w), preferably in a weight ratio of between 1:1 to 15:1 (w/w), more preferably in between 5:1 to 10:1 (w/w); and one or more pharmaceutically acceptable excipient.

In one embodiment of the invention, crospovidone is present in an amount of between 1.0 to 30.0 % by weight, preferably in an amount of 5.0 to 25.0 % by weight, more preferably it is 10.0 to 20.0 % by wieght of the total formulation; sodium stearyl fumarate is present in an amount of between 0.10 to 10.0 % by weight, preferably it is 1.0 to 5.0 % by weight of total formulation. According to these embodiments the formulation disintegrates in oral cavity in less than 60 seconds, preferably in less than 30 seconds, more preferably in less than 20 seconds.

In another embodiment, the orally disintegrating tablet formulation comprises mirtazapine or a pharmaceutically acceptable salt thereof in an amount of 0.10 to 10.0 % by weight, preferably it is 1.0 to 5.0 % by weight of total tablet formulation.

Another object of the present invention is to provide an orally disintegrating tablet formulation of mirtazapine or a pharmaceutically acceptable salt thereof comprising antioxidants which are selected from the group comprising methyl paraben and propyl paraben and their salts (such as sodium, potassium), sodium benzoate, citric acid, benzoic acid, butylated hydroxytoluene (BHT), butylated hydroxyanisole (BHA) and the like and mixtures thereof. In this present invention, the suitable antioxidans are preferably butylated hydroxytoluene (BHT) and butylated hydroxyanisole (BHA). In one aspect, the preservative content may present in an amount of about from 0,01 to 5.0%, preferably about from 0.5 to 2.0% by weight of total composition. Using these antioxidants provide a stable formulation throughout the shelf-life and avoid using other complex excipients (such as polymers, HPMC, polymethacrylate ....etc) cause time consuming and complex manufacturing steps.

In a further embodiment the orally disintegrating tablet formulation of mirtazapine or a pharmaceutically acceptable salt thereof comprises one or more pharmaceutically acceptable excipients other than crospovidone, sodium stearyl fumarate and antioxidants wherein the one or more pharmaceutically acceptable excipients are selected from the group comprising diluents, sweetners and flavouring agents.

Suitable diluents are selected from the group comprising mannitol, polysaccharides, primarily microcrystalline cellulose, dibasic calcium phosphate dihydrate, lactose, sugars, starch, inorganic salts, primarily calcium salts and the like and mixtures thereof; preferably the diluents are mannitol and microcrystalline cellulose.

In contrast to prior art formulations it is not necessary to use a highly-compressible quality, such as spray-dried mannitol which is also more expensive than the mannitol. According to this embodiment of the invention, the orally disintegrating tablet formulation of mirtazapine or a pharmaceutically acceptable salt thereof comprises mannitol, in an amount of between 5.0 to 85.0 % by weight, preferably it is 35.0 to 70.0 % by weight, more preferably it is 40.0 to 60.0 % by weight of total formulation.

In a further embodiment, microcrystalline cellulose is present in an amount of between 5.0 to 85.0 % by weight, preferably it is 10.0 to 50.0 % by weight of total formulation.

Another object of the present invention is to develop orally disintegrating compositions having optimal mechanical strength. The present invention addresses this need and discloses formulations that rapidly disintegrate in the oral cavity. These tablet compositions have a pleasant mouth feel and good mechanical strength. These tablets are robust (e.g., low friability, adequate hardness) enough to be processed in high speed tableting machines and shipped in low cost packages, and at the same time retain rapid disintegration or dissolution properties. These orally disintegrating compositions are bioavailable in correspondence with the conventional solid dosage formulations and stable throughout the shelf-life.

It has surprisingly been found that the specific combination of mannitol and microcrystalline cellulose with the active ingredient mirtazapine or a pharmaceutically acceptable salt thereof results a synergistic effect over the mechanical strength (such as; hardness and friability) and disintegration time of the orally disintegrating tablet formulation. According to this embodiment, the weight ratio of mannitol to microcrystalline cellulose is in the range of between 1:10 and 20:1 (w/w), preferably it is in the range of between 1:5 and 10:1 (w/w), more preferably it is in the range of between 1:2 to 5:1 (w/w); said amount makes it possible to significantly improve compressibility, reduce friability and achieve a substantial reduction in disintegration time. Higher quantities may have negative mechanichal strength of the formulation and lower quantities may worsen the disintegration time.

According to this object of the present invention, the hardness of the orally disintegrating tablet is between 5 N to 100 N, preferably it is between 20 N to 50 N; and the friabilite of the orally disintegrating tablet is less than 1.0%.

Suitable sweetners are selected from the group comprising sucralose, fructose, glucose, sorbitol, saccharin, sodium cyclamate, acesulfame-K and the like and mixtures thereof; preferably the sweetner is sucralose. The selection of sucralose as sweetner has lots of advantages such as improving patient compliance. In prior art, it is know that aspartame is used mostly as sweetner but contradictory to the prior art we have found that the effect of sucralose as a sweetner in this formulation, not only helped to improve its taste but also increased the efficacy and the conveniency of the formulation because of its positive effects over the glycemic index. There are lots of disadvantages about aspartame and it has a limited usage if you have to use it every day and also there are several incompatibilities reported in literature and safety problems Thus, sucralose has an important role in this aspect and even if it is used in low amounts it has a synergistic taste improvement with mannitol which is also very important issue in orally disintegrating tablet formulations. According to this object of the present invention sucralose is present in an amount of between 0.01 to 3.00 % by weight, preferably it is 0.05 to 1.50 % by weight of total formulation.

Suitable flavouring agents may comprise but not limited to fruit flavours such as orange, banana, strawberry, cherry, wild cherry, lemon; and other flavours such as cardamom, anise, peppermint, menthol, vanillin and ethyl vanillin and the like and mixtures thereof; preferably the flavouring agent is fruit flavour such as orange. In one aspect, flavouring agent content is present in an amount of from 0.01 to 5.0%, preferably from 0.10 to 2.0% by weight of total composition.

As it is mentioned above, to develop orally disintegrating compositions are difficult because of several different reasons. A satisfied orally disintegrating dosage form needs to meet number of requirements. Firstly, it has to disintegrate in the oral cavity rapidly. Moreover, a premature release in the mouth could also lead to problems due to the often unpleasant taste of the active ingredient. Besides, these compositions should be very porous and should not be very hard. These porous compositions tend to be very sensitive to humidity. As a consequence, they may have some stability problems.

However, in order to be pharmacologically acceptable, orally disintegrating compositions must be palatable, e.g. have acceptable organoleptic properties such as good taste and mouthfeel, because orally disintegrating compositions are designed to disintegrate in the oral cavity of the patient rapidly without remaning substantial amounts of the active ingredient. In addition, the orally disintegrating formulations must also provide acceptable pharmacokinetics and bioavailability to provide the desired therapeutic effect. Conversely, components of the formulation that promote rapid release may result in undesirable taste or mouthfeel properties. Finally, any orally disintegrating composition with suitable organoleptic and pharmacokinetic properties must also be manufactured at commercially useful rates and yields.

To fulfill all these requirements the formulation for a specific drug needs to be adapted in particular by a careful selection of the excipients used. However, the excipients selected may lead to formulations which are not bioavailable to the corresponding conventional dosage forms. Thus, they have to be chosen very carefully.

In this present invention, to minimize the disintegration time and maximise the mechanical resistance of the tablets of this invention, this orally disintegrating tablet formulaiton has been designed, consisting the followings:
a. 0.10 to 10.0% by weight of mirtazapine hemihydrate,
b. 5.0 to 85.0% by weight of mannitol,
c. 1.0 to 30.0% by weight of crospovidone,
d. 5.0 to 85.0% by weight of microcrystalline cellulose,
e. 0.01 to 3.00% by weight of sucralose,
f. 0.01 to 5.0% by weight of orange flavour,
g. 0.1 to 10.0 % by weight of sodium stearyl fumarate,
h. 0.01 to 2.00% by weight of butylated hydroxyanisole (BHA)
i. 0.01 to 2.00% by weight of butylated hydroxytoluene (BHT)

Another object of the invention is to provide a simple and time-saving process for the preparation of orally disintegrating tablet formulation of mirtazapine or a pharmaceutically acceptable salt thereof. According to this object of the invention, the preferred process of the present invention for preparing the orally disintegrating tablet formulation is wet granulation which comprises the following steps;
a. dissolving butylated hydroxyanisole and butylated hydroxytoluene in an alcohol to form a solution,
b. sieving mirtazapine and two-thirds(2/3) of mannitol and mixing them,
c. granulating the mixture with the solution,
d. sieving and drying the wet granules and milling the dried granules,
e. adding the rest of the mannitol, crospovidone, microcrystalline cellulose, sucralose, flavour and mixing them,
f. adding sodium stearyl fumarate to this mixture and blending them until obtaining a homogenous powder mixture,
g. compressing the blended mixture to form tablets.

In a further aspect, the present invention shows that it is possible to have a significant influence on the disintegration rate of the tablet by modifying the dimensions and shape of the tablet. In general, as the tablet becomes thinner and have higher porosity, the orally disintegrating composition will be weakened faster when it contacts with saliva, because the disintegration process is produced after wetting all the surface of the tablet via capillary action. Also, any shape which maximizes the contact surface with the saliva may produce a significant reduction in disintegration time.

The preferred shape of the orally disintegrating tablet composition of this invention may have a shape of a disk, circle, round, sphere, donut, bar, polygon, ellipse and the like. The preffered shape of the tablet is a round shape.

This invention is further defined by reference to the following examples. Although the examples are not intended to limit the scope of the present invention, it should be considered in the light of the description detailed above. It will be apparent to those skilled in the art that many modifications, both to materials and methods, may be practiced without departing from the scope of the invention.

### Examples

### Example 1 - Orally disintegrating mirtazapine hemihydrate tablets

| **Ingredients** | **Amount** **(%)** |
|---|---|
| Mirtazapine hemihydrate | 4.84 |
| Sucralose | 0.20 |
| Orange flavour | 1.00 |
| Microcrystallie cellulose (Avicel CE-15) | 5.00 |
| Mannitol | 48.13 |
| Microcrystallie cellulose (Avicel pH-301) | 23.77 |
| Crospovidone (Kollidon CL) | 15.00 |
| butylated hydroxyanisole (BHA) | 0.03 |
| butylated hydroxytoluene (BHT) | 0.03 |
| Sodium stearyl fumarate | 2.00 |
| Total tablet weight | 100.00 |

This formulation is prepared by wet granulation as described in detail above. Firstly, butylated hydroxyanisole and butylated hydroxytoluene is dissolved in an alcohol to form a solution, then mirtazapine and two-thirds (2/3) of mannitol is sievied and mixed. This mixture is granulated with the solution and the wet granules are sieved and dried, then the fried granules are milled. The rest of the mannitol, crospovidone, microcrystalline cellulose, sucralose and orange flavour is added and then sodium stearyl fumarate is added to this mixture and blended together until obtaining a homogenous powder mixture. Finally, the blended mixture is compressed to form tablets.

### Hardness, Friability and Disintegration Test Results

According to standardized methods and equipment for testing friability, hardness and disintegrating time have been provided in European Pharmacopeia. The orally disintegrating tablet formulations of the invention (Ex.1) is tested according to these methods. As it is seen in the Table 1, the hardness of the tablets is quite sufficient to allow easy and convenient removal from the package without breaking the dosage unit. These orally disintegrating tablets are hard enough to be handled and packaged like conventional tablets. They are compressed to a hardness of 20 - 50 Newton and possess a friability of less than 1%. The disintegrating times are acceptable and the taste and mouthfeel of the tablets are good. The reference example is also tested under the same conditions, they are compressed to a hardness of 20 - 50 Newton. Also mirtazapine hemihydrate and total impurity contents are tested according to the methods which are developed and validated. The excipients of the reference tablet are same with the one with Example 1 except sodium stearyl fumarate and additionally magnesium stearate and colloidal silicon dioxide is added to this referenece.

**Table 1**

| | **Example 1** | **Reference example** |
|---|---|---|
| **Harndness (Newton)** | 38 | 38 |
| **Friability (%)** | 0,48 | 1.05 |
| **Disintegration time (sec)** | 12 | 20 |
| **Mirtazapine hemihydrate content (mg) (±14.25-15,75)** | 14.925 | 14.765 |
| **Total impurity (±%1.0)** | 0.051 | 0.186 |

### Dissolution Results

The orally disintegrating tablet formulation of this invention (Example 1) and the reference example are tested under the test conditions of 0.1 N HCl in 900 ml using a USP paddle method rotating at 50 rpm (USP Apparatus II). Results are shown in Table 2.

**Table 2**

| **Time** **(minutes)** | **Example 1** **Mirtazapine content (%)** | **Reference example** **Mirtazapine content (%)** |
|---|---|---|
| **2** | 88,95 | 72.57 |
| **4** | 99,45 | 100.37 |
| **6** | 101,63 | 103.50 |
| **10** | 101.54 | 104.37 |
| **15** | 101.78 | 104.80 |

### Stability Results

The orally disintegrating tablet formulation of this invention may have a long-term shelf-life of 24 months or more at ambient temperature in its original packaging. The stability test results of Example 1 are shown in Table 3.

**Table 3 (Example 1)**

| **Storage conditions** **(°C/% relative humidty)** | **Storage time** **(months)** | **Mirtazapine content** **(mg)** | **Total Impurity** **(%)** | **Disintegration time** **(second)** | **Hardness** **(Newton)** |
|---|---|---|---|---|---|
| 25°C±2°C | 0 | 14,925 | 0.051 | 12 | 38 |
| 25°C±2°C / %60±%5 | 3 | 14,625 | 0.058 | 11 | 37 |
| 30°C±2°C/ %65±%5 | 3 | 14,520 | 0.067 | 10 | 32 |
| 40°C±2°C/ %75±%5 | 3 | 14,625 | 0.085 | 10 | 27 |

## Claims

1. A silicon dioxide free orally disintegrating tablet formulation of mirtazapine or a pharmaceutically acceptable salt thereof comprising crospovidone and sodium stearyl fumarate in a weight ratio of between 1:25 to 25:1 (w/w) and one or more pharmaceutically acceptable excipient.

2. The orally disintegrating tablet formulation of mirtazapine or a pharmaceutically acceptable salt thereof according to claim 1, further not comprising magnesium stearate.

3. The orally disintegrating tablet formulation of mirtazapine or a pharmaceutically acceptable salt thereof according to claim 1 and 2, comprising antioxidants which are selected from the group comprising butylated hydroxyanisole (BHA), butylated hydroxytoluene (BHT), ascorbic acid, beta-Carotene, alpha-tocopherol, propyl gallate, gentisic acid sodium ascorbate, sodium bisulfite, sodium metabisulfite, monothioglycero, cysteine, thioglycolate sodium; preferably the antioxidants are butylated hydroxyanisole (BHA) and butylated hydroxytoluene (BHT).

4. The orally disintegrating tablet formulation of mirtazapine or a pharmaceutically acceptable salt thereof according to claim 1, wherein mirtazapine or a pharmaceutically acceptable salt is present in an amount of 0.10 to 10.0 % by weight, preferably it is 1.0 to 6.0 % by weight of total formulation.

5. The orally disintegrating tablet formulation of mirtazapine or a pharmaceutically acceptable salt thereof according to claim 1, wherein crospovidone is present in an amount of between 1.0 to 30.0 % by weight, preferably it is 5.0 to 25.0 % by weight, more preferably it is 10.0 to 20.0 % by weight of the total formulation.

6. The orally disintegrating tablet formulation of mirtazapine or a pharmaceutically acceptable salt thereof according to claim 1, wherein sodium stearyl fumarate is present in an amount of between 0.10 to 10.0 % by weight, preferably it is 1.0 to 5.0 % by weight of total formulation.

7. The orally disintegrating tablet formulation of mirtazapine or a pharmaceutically acceptable salt thereof according to claims 5 and 6, wherein the weight ratio of crospovidone and sodium stearyl fumarate is preferably between 1:1 to 15:1 (w/w), more preferably it is 5:1 to 10:1 (w/w).

8. The orally disintegrating tablet formulation of mirtazapine or a pharmaceutically acceptable salt thereof according to claims 1 to 7, wherein the composition disintegrates in oral cavity in less than 60 seconds, preferably in less than 30 seconds, more preferably less than 20 seconds.

9. The orally disintegrating tablet formulation of mirtazapine or a pharmaceutically acceptable salt thereof according to claims 1 to 7, wherein the hardness of the tablet is between 5 N to 100 N, preferably it is between 20 N to 50 N.

10. The orally disintegrating tablet formulation of mirtazapine or a pharmaceutically acceptable salt thereof according to claims 1 to 7, wherein the friability of the tablet is less than 1.0%.

11. The orally disintegrating tablet formulation of mirtazapine or a pharmaceutically acceptable salt thereof according to claim 1, wherein the one or more pharmaceutically acceptable excipients are selected from the group comprising diluents, sweetners and flavouring agents.

12. The orally disintegrating tablet formulation of mirtazapine or a pharmaceutically acceptable salt thereof according to claim 11, wherein the diluents are preferably mannitol and microcrystalline cellulose and the weight ratio of mannitol to microcrystalline cellulose is in the range of between 1:10 to 20:1 (w/w), preferably it is 1:5 to 10:1 (w/w), more preferably it is 1:2 to 5:1 (w/w).

13. The orally disintegrating tablet formulation of mirtazapine or a pharmaceutically acceptable salt thereof according to claim 11, wherein the sweetner is preferably sucralose and sucralose is present in an amount of between 0.01 to 3.00 % by weight, preferably it is 0.05 to 1.50 % by weight of total formulation.

14. The orally disintegrating tablet formulation of mirtazapine or a pharmaceutically acceptable salt thereof according to any preceding claim, consisting;
a. 0.10 to 10.0% by weight of mirtazapine hemihydrate,
b. 5.0 to 85.0% by weight of mannitol,
c. 1.0 to 30.0% by weight of crospovidone,
d. 5.0 to 85.0% by weight of microcrystalline cellulose,
e. 0.01 to 3.00% by weight of sucralose,
f. 0.01 to 5.0% by weight of orange falvour,
g. 0.1 to 10.0 % by weight of sodium stearyl fumarate,
h. 0.01 to 2.00% by weight of butylated hydroxyanisole (BHA)
i. 0.01 to 2.00% by weight of butylated hydroxytoluene (BHT)

15. A process for preparing orally disintegrating tablet formulation of mirtazapine or a pharmaceutically acceptable salt thereof according to any preceding claim comprising;
a. dissolving butylated hydroxyanisole and butylated hydroxytoluene in an alcohol to form a solution,
b. sieving mirtazapine and two-thirds(2/3) of mannitol and mixing them,
c. granulating the mixture with the solution,
d. sieving and drying the wet granules and milling the dried granules,
e. adding the rest of the mannitol, crospovidone, microcrystalline cellulose, sucralose, flavour and mixing them,
f. adding sodium stearyl fumarate to this mixture and blending them until obtaining a homogenous powder mixture,
g. compressing the blended mixture to form tablets.

## Patentansprüche

1. Eine siliziumdioxidfreie sich oral zerfallende Tablettenzusammensetzung von Mirtazapin oder einem pharmazeutisch akzeptablen Salz umfassend Crospovidon und Natriumstearylfumarat in einem Gewichtsverhältnis zwischen 1:25 bis 25:1 (w/w) und ein oder mehrere pharmazeutisch akzeptablen Hilfsstoffe.

2. Oral zerfallende Tablettenzusammensetzung von Mirtazapin oder einem davon pharmazeutisch akzeptablen Salz gemäß Anspruch 1, des Weiteren kein Magnesiumstearat umfassen.

3. Oral zerfallende Tablettenzusammensetzung von Mirtazapin oder einem davon pharmazeutisch akzeptablen Salz gemäß Anspruch 1 und 2 umfassend Antioxidantien, welche ausgewählt sind aus der Gruppe umfassend butyliertes Hydroxyanisol (BHA), butyliertes Hydoxytoluen (BHT), Ascorbinsäure, beta-Carotin, alpha-Tocopherol, Propylgalat, Gentisinsäure, Natriumascorbat, Natriumbisulfit, Natriummetabisulfit, Monothioglycerol, Cystein, Thioglykolatnatrium; bevorzugte Antioxidatien sind butyliertes Hydroxyanisol (BHA) und butyliertes Hydroxytoluen (BHT).

4. Oral zerfallende Tablettenzusammensetzung von Mirtazapin oder einem davon pharmazeutisch akzeptablen Salz gemäß Anspruch 1, wobei Mirtazapin oder ein davon pharmazeutisch akzeptables Salz in einer Menge von 0,1 bis 10 Gew.%, bevorzugt 1,0 bis 6,0 Gew.% der Gesamtzusammensetzung vorhanden ist.

5. Oral zerfallende Tablettenzusammensetzung von Mirtazapin oder einem davon pharmazeutisch akzeptablen Salz gemäß Anspruch 1, wobei Crospovidon in einer Menge zwischen 1,0 bis 30,0 Gew.%, bevorzugt 5,0 bis 25 Gew.%, noch bevorzugter zwischen 10,0 bis 20,0 Gew.% in Bezug auf die Gesamtzusammensetzung vorhanden ist.

6. Oral zerfallendeTablettenzusammensetzung von Mirtazapin oder einem davon pharmazeutisch akzeptablen Salz gemäß Anspruch 1, wobei Natriumstearylfumarat in einer Menge zwischen 0,1 bis 10 Gew.%, bevorzugt zwischen 1,0 bis 5,0 Gew.% der Gesamtzusammensetzung vorhanden ist.

7. Oral zerfallende Tablettenzusammensetzung von Mirtazapin oder einem davon pharmazeutisch akzeptablen Salz gemäß der Ansprüche 5 und 6, wobei das Gewichtsverhältnis von Crospovidon und Natriumstearylfumarat bevorzugt zwischen 1:1 bis 15:1 (w/w), noch bevorzugter zwischen 5:1 bis 10:1 (w/w) beträgt.

8. Oral zerfallende Tablettenzusammensetzung von Mirtazapin oder einem davon pharmazeutisch akzeptablen Salz gemäß den Ansprüchen 1 bis 7, wobei sich die Zusammensetzung in der Mundhöhle in weniger als 60 Sekunden, bevorzugt in weniger als 30 Sekunden, noch bevorzugter in weniger als 20 Sekunden auflöst.

9. Oral zerfallende Tablettenzusammensetzung von Mirtazapin oder einem davon pharmazeutisch akzeptablen Salz gemäß den Ansprüchen 1 bis 7, wobei die Härte der Tabletten zwischen 5 N bis 100 N, bevorzugt zwischen 20 N bis 50 N ist.

10. Oral zerfallende Tablettenzusammensetzung von Mirtazapin oder einem davon pharmazeutisch akzeptablen Salz gemäß Anspruch 1 bis 7, wobei die Zerreibbarkeit der Tabletten weniger als 1 % ist.

11. Oral zerfallende Tablettenzusammensetzung von Mirtazapin oder einem davon pharmazeutisch akzeptablen Salz gemäß Anspruch 1, wobei die ein oder mehreren pharmazeutisch akzeptablen Hilfsstoffe ausgewählt sind aus der Gruppe umfassend Verdünnungsmittel, Süßstoffe und Geschmacksstoffe.

12. Oral zerfallende Tablettenzusammensetzung von Mirtazapin oder einem davon pharmazeutisch akzeptablen Salz gemäß Anspruch 11, wobei die Verdünnungsmittel bevorzugt Manitol und mikrokristalline Zellulose sind und das Gewichtsverhältnis von Manitol zu mikrokristalliner Zellulose in einem Bereich zwischen 1:10 bis 20:1 (w/w), bevorzugt zwischen 1:5 bis 10:1 (w/w), noch bevorzugter zwischen 1:2 bis 5:1 (w/w) beträgt.

13. Oral zerfallende Tablettenzusammensetzung von Mirtazapin oder einem davon pharmazeutisch akzeptablen Salz gemäß Anspruch 11, wobei der Süßstoff bevorzugt Sucralose ist und Sucralose in einer Menge zwischen 0,01 bis 3,00 Gew.%, bevorzugt zwischen 0,05 bis 1,5 Gew.% der Gesamtzusammensetzung vorhanden ist.

14. Oral zerfallende Tablettenzusammensetzung von Mirtazapin oder einem davon pharmazeutisch akzeptablen Salz gemäß einem der vorhergehenden Ansprüche bestehend aus
a. 0,1 bis 10,0 Gew.% Mirtazapinhemihydrat,
b. 5,0 bis 85,0 Gew.% Manitol,
c. 1,0 bis 30,0 Gew.% Crospovidon,
d. 5,0 bis 85,0 Gew.% mikrokristalline Zellulose,
e. 0,01 bis 3,0 Gew.% Sucralose,
f. 0,01 bis 5,0 Gew.% Orangengeschmacksstoff,
g. 0,1 bis 10,0 Gew.% Natriumstearylfumarat,
h. 0,01 bis 2,0 Gew.% butyliertes Hydroxyanisol (BHA),
i. 0,01 bis 2,0 Gew.% butyliertes Hydroxytoluen (BHT).

15. Ein Verfahren zur Herstellung einer oral zerfallenden Tablettenzusammensetzung von Mirtazapin oder einem davon pharmazeutisch akzeptablen Salz gemäß einem der vorhergehenden Ansprüche umfassend:
a. Auflösen von butyliertem Hydroxyanisol und butyliertem Hydroxytoluen in einem Alkohol unter Ausbildung einer Lösung,
b. Sieben von Mirtazapin und zwei Dritteln (2/3) von Manitol und Vermischen derselben,
c. Granulieren der Mischung mit der Lösung,
d. Sieben und Trocknen der nassen Granulate und Mahlen der getrockneten Granulate,
e. Zufügen des Restes an Manitol, Crospovidon, mikrokristalliner Zellulose, Sucralose, Geschmacksstoff und Vermischen derselben,
f. Zufügen von Natriumstearylfumarat zu dieser Mischung und Vermischen bis eine homogene Pulvermischung erhalten wird,
g. Verpressen der Mischung unter Ausbildung von Tabletten.

## Revendications

1. Formulation de mirtazapine en comprimé à désintégration orale sans dioxyde de silicium ou sel pharmaceutiquement acceptable de celle-ci comprenant de la crospovidone et du fumarate de stéaryle sodique dans un rapport pondéral entre 1/25 et 25/1 (p/p) et un ou plusieurs excipients pharmaceutiquement acceptables.

2. Formulation de mirtazapine en comprimé à désintégration orale ou sel pharmaceutiquement acceptable de celle-ci selon la revendication 1, ne comprenant en outre pas de stéarate de magnésium.

3. Formulation de mirtazapine en comprimé à désintégration orale ou sel pharmaceutiquement acceptable de celle-ci selon les revendications 1 et 2, comprenant des antioxydants qui sont sélectionnés dans le groupe comprenant l'hydroxyanisole butylé (BHA), l'hydroxytoluène butylé (BHT), l'acide ascorbique, le bêta-carotène, l'alpha-tocophérol, le gallate de propyle, l'acide gentisique, l'ascorbate de sodium, le bisulfite de sodium, le métabisulfite de sodium, le monothioglycéro, la cystéine, le thioglycolate de sodium ; de préférence les antioxydants sont l'hydroxyanisole butylé (BHA) et l'hydroxytoluène butylé (BHT).

4. Formulation de mirtazapine en comprimé à désintégration orale ou sel pharmaceutiquement acceptable de celle-ci selon la revendication 1, où la mirtazapine ou un sel pharmaceutiquement acceptable est présent en une quantité de 0,10 à 10,0 % en poids, de préférence qui s'élève à 1,0 à 6,0 % en poids de la formulation totale.

5. Formulation de mirtazapine en comprimé à désintégration orale ou sel pharmaceutiquement acceptable de celle-ci selon la revendication 1, où de la crospovidone est présente en une quantité entre 1,0 et 30,0 % en poids, de préférence qui s'élève à 5,0 à 25,0 % en poids, plus préférablement qui s'élève à 10,0 à 20,0 % en poids de la formulation totale.

6. Formulation de mirtazapine en comprimé à désintégration orale ou sel pharmaceutiquement acceptable de celle-ci selon la revendication 1, où le fumarate de stéaryle sodique est présent en une quantité entre 0,10 et 10,0 % en poids, de préférence qui s'élève à 1,0 à 5,0 % en poids de la formulation totale.

7. Formulation de mirtazapine en comprimé à désintégration orale ou sel pharmaceutiquement acceptable de celle-ci selon les revendications 5 et 6, où le rapport pondéral entre la crospovidone et le fumarate de stéaryle sodique est de préférence entre 1/1 et 15/1 (p/p), plus préférablement il est de 5/1 à 10/1 (p/p).

8. Formulation de mirtazapine en comprimé à désintégration orale ou sel pharmaceutiquement acceptable de celle-ci selon les revendications 1 à 7, où la composition se désintègre dans une cavité orale en moins de 60 secondes, de préférence en moins de 30 secondes, plus préférablement en moins de 20 secondes.

9. Formulation de mirtazapine en comprimé à désintégration orale ou sel pharmaceutiquement acceptable de celle-ci selon les revendications 1 à 7, où la dureté du comprimé se situe entre 5 N et 100 N, de préférence elle se situe entre 20 N et 50 N.

10. Formulation de mirtazapine en comprimé à désintégration orale ou sel pharmaceutiquement acceptable de celle-ci selon les revendications 1 à 7, où la friabilité du comprimé est inférieure à 1,0 %.

11. Formulation de mirtazapine en comprimé à désintégration orale ou sel pharmaceutiquement acceptable de celle-ci selon la revendication 1, où les un ou plusieurs excipients pharmaceutiquement acceptables sont sélectionnés dans le groupe comprenant les diluants, les édulcorants et les agents de saveur.

12. Formulation de mirtazapine en comprimé à désintégration orale ou sel pharmaceutiquement acceptable de celle-ci selon la revendication 11, où les diluants sont de préférence le mannitol et la cellulose microcristalline et le rapport pondéral entre le mannitol et la cellulose microcristalline se situe dans la plage entre 1/10 et 20/1 (p/p), de préférence il est de 1/5 à 10/1 (p/p), plus préférablement il est de 1/2 à 5/1 (p/p).

13. Formulation de mirtazapine en comprimé à désintégration orale ou sel pharmaceutiquement acceptable de celle-ci selon la revendication 11, où l'édulcorant est de préférence le sucralose et le sucralose est présent en une quantité entre 0,01 et 3,00 % en poids, de préférence qui s'élève à 0,05 à 1,50 % en poids de la formulation totale.

14. Formulation de mirtazapine en comprimé à désintégration orale ou sel pharmaceutiquement acceptable de celle-ci selon l'une quelconque des revendications précédentes, comprenant :
a. de 0,10 à 10,0 % en poids d'hémihydrate de mirtazapine,
b. de 5,0 à 85,0 % en poids de mannitol,
c. de 1,0 à 30,0 % en poids de crospovidone,
d. de 5,0 à 85,0 % en poids de cellulose microcristalline,
e. de 0,01 à 3,00 % en poids de sucralose,
f. de 0,01 à 5,0 % en poids d'arôme d'orange,
g. de 0,1 à 10,0 % en poids de fumarate de stéaryle sodique,
h. de 0,01 à 2,00 % en poids d'hydroxyanisole butylé (BHA),
i. de 0,01 à 2,00 % en poids d'hydroxytoluène butylé (BHT).

15. Procédé de préparation d'une formulation de mirtazapine en comprimé à désintégration orale ou d'un sel pharmaceutiquement acceptable de celle-ci selon l'une quelconque des revendications précédentes, comprenant :
a. la dissolution de l'hydroxyanisole butylé et de l'hydroxytoluène butylé dans un alcool pour former une solution,
b. le tamisage de la mirtazapine et des deux tiers (2/3) du mannitol et leur mélange,
c. la granulation du mélange avec la solution,
d. le tamisage et le séchage des granules humides et le broyage des granules séchés,
e. l'addition du reste de mannitol, de crospovidone, de cellulose microcristalline, de sucralose, d'arôme et leur mélange,
f. l'addition de fumarate de stéaryle sodique à ce mélange et leur brassage jusqu'à obtention d'un mélange poudreux homogène,
g. la compression du mélange brassé pour former des comprimés.
